Europäisches Patentamt

European Patent Office

Office européen des brevets

(19)

(11) Publication number: **0 096 586 B1**

# EUROPEAN PATENT SPECIFICATION

(45) Date of publication of patent specification: **10.04.91**

(51) Int. Cl.⁵: **C12N 15/63**, C12N 1/20, C12P 21/00

(21) Application number: **83303293.1**

(22) Date of filing: **07.06.83**

(54) **Temperature sensitive multicopy plasmids and organisms containing the same, their production and use in protein production.**

(30) Priority: **08.06.82 US 386348**

(43) Date of publication of application:
**21.12.83 Bulletin 83/51**

(45) Publication of the grant of the patent:
**10.04.91 Bulletin 91/15**

(84) Designated Contracting States:
**AT BE CH DE FR GB IT LI LU NL SE**

(56) References cited:
**EP-A- 0 003 062**
**EP-A- 0 060 045**
**EP-A- 0 105 554**

**PROCEEDINGS OF THE NATIONAL ACADEMY OF SCIENCES USA, vol. 79,4th June 1982, pages 3570-3574; E.M.WONG et al.: "Temperature-sensitive copy number mutants of CoIE1 are located in an untranslated region of the plasmid genome"**

**NATURE, vol. 290, 19th March 1981, pages 264-267; A.R.STUITJE et al.: "Identification of mutations affecting replication control of**

plasmid Clo DF13"

Proceedings of National Academy of Science, Gelfand et al., vol. 75, pp 5869

Cell, Heffron et al., 1979, 18, 1153

(73) Proprietor: **CETUS CORPORATION**
**1400 Fifty-Third Street**
**Emeryville California 94608(US)**

(72) Inventor: **Gelfand, David Harow**
**6208 Chelton Drive**
**Oakland California 94611(US)**
Inventor: **Strange, Carolyn Jan**
**35860 Ashton Place**
**Fremont, California 94703(US)**
Inventor: **Watson, Robert Malcolm**
**1819 Berkeley Way**
**Berkeley California 94703(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

## Description

This invention relates generally to the art of genetic engineering and, more particularly, to improved plasmids by which copy number may be controlled by temperature, and to improved strains carrying such plasmids. Organisms containing such plasmids have been deposited with the American Type Culture Collection (ATCC 39142).

Naturally occurring plasmids exist in host cells in a stable and characteristic copy number per host chromosome. This copy number is typically fairly constant although some variation may exist from host to host and may be affected by certain physiological phenomena such as culture growth rate, growth history, etc.

One of the factors which govern the copy number of a given plasmid in a host cell line is the plasmid's own replication system. Plasmid mutants have been isolated in which the copy number is far higher than that typically found in naturally occurring or wild-type organisms. Although not fully understood, such high copy number mutants are believed to exist as a result of an altered repressor mechanism. Stable high copy number mutants are shown and described in European Patent No. 0013830.

Assuming that the expression mechanisms of the host cells are viable, the existence of high copy number plasmids can also provide a corresponding increase in the production of proteins encoded by genes on those plasmids. Thus, the use of high copy number plasmids in genetic engineering affords a means by which the product yield of heterologous genes carried by such plasmids may be increased.

Under some circumstances, the production of large amounts of protein product of a heterologous gene can have a deleterious affect on the host organism. Under such circumstances, the ability to produce large amounts of the desired protein product may be impaired, particularly in large scale industrial fermentation processes. Thus, it is desirable to find a means for controlling the level of production of heterologous gene products in genetically engineered microorganisms.

Expression promoter mechanisms are known which may be controlled, for example by the addition or depletion of a particular substance. Such a promoter mechanism may be employed in connection with high copy number mutants in order to delay production of a heterologous gene product until the host cultures have had an opportunity to grow to a desired maturity. However, on an industrial scale, such method of expression control may be undesirable because of the necessity of providing large amounts of inducer to the culture, or removing large amounts of inducer from the culture, at the desired time. Moreover, host cells in which high copy number plasmids exist are essentially "sick" and may not thrive as well as hoped under industrial conditions.

Plasmids have been described whose replication systems are temperature sensitive. Thus, the plasmids may exist in low copy number at one temperature, and at another temperature may replicate rapidly to a very high or even a runaway copy number. Such plasmids afford a desirable technique for switching on the production of heterologous gene products, not through the expression mechanism as above described, but through the replication mechanism. Thus, host cell cultures may be grown under conditions designed to foster growth without experiencing an unusually high production of the heterologous gene product encoded for by the genetically engineered plasmid. Once a desired growth level is reached, the production of the heterologous gene product may be greatly increased or switched on by altering the temperature to cause high copy replication of the temperature sensitive plasmids.

Although several temperature sensitive copy number plasmids are known in the prior art, such plasmids may not be desirable in certain circumstances, particularly in large scale industrial applications. This is because many of such prior art plasmids may not be stable, may require enriched media for proper amplification, or may require extensive growth of the host microorganism in log phase before satisfactory amplification may be achieved with temperature shift. In addition, some of the prior art plasmids which are temperature sensitive copy number plasmids require repeated growth/dilution phases before amplification may be achieved.

According to the invention there is provided a stable recombinant plasmid having the following characteristics:

the plasmid comprises a region derived from a colicin El (Col El)-type plasmid, wherein said region causes intracellular replication of the plasmid to undergo an increase upon an increase in temperature, wherein said increase in replication is independent of protein synthesis and is RNA-controlled;

the plasmid lacks an active transposon 3 (Tn3);

the plasmid comprises two regions each encoding drug resistance to a different drug, one of the regions containing a restriction enzyme site positioned such that insertion of a DNA sequence into said site causes loss of the respective drug resistance; and

after insertion of a DNA sequence into the plasmid expressibly encoding a foreign polypeptide the resulting plasmid is stable during expression of the encoded foreign polypeptide when the copy number of the plasmid has been increased.

FIGURE 1 is a restriction map of the plasmid designated pOP9 used in the preparation of the plasmid of the invention;

FIGURE 2 is a restriction map of the plasmid designated as pEW27 used in the preparation of a plasmid of the invention; and

FIGURE 3 is a restriction map of a plasmid designated pCS3 constructed in accordance with the invention.

Very generally, the stable high copy number plasmids of the invention have ColEI type parentage and undergo substantial intracellular replication in a host upon a substantial increase in temperature.

More particularly, the plasmids of the invention are produced by incorporating portions of known ColEI derivative plasmids to produce the particularly exceptional characteristics of the plasmids of the invention. The plasmids may be relatively small (less than six kilobases) and have a fragment containing mutations which provide the temperature sensitive copy number characteristics. Naturally, the plasmids are also provided with a suitable site for the insertion of heterologous DNA and either contain suitable promoters to provide expression of such heterologous DNA, or provide for the insertion of such promoters along with the heterologous DNA.

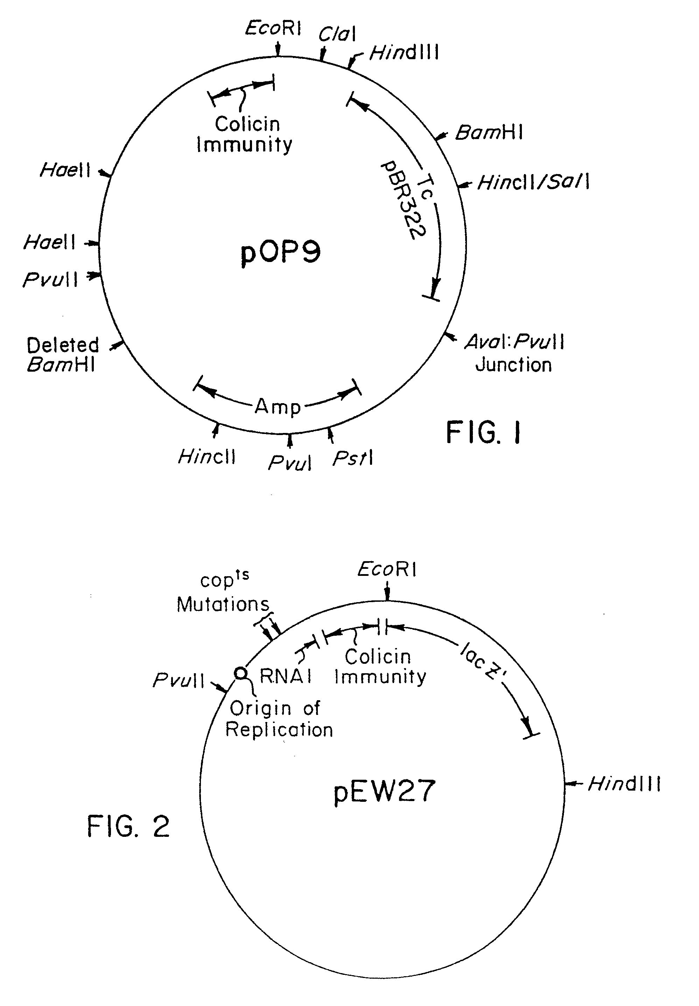

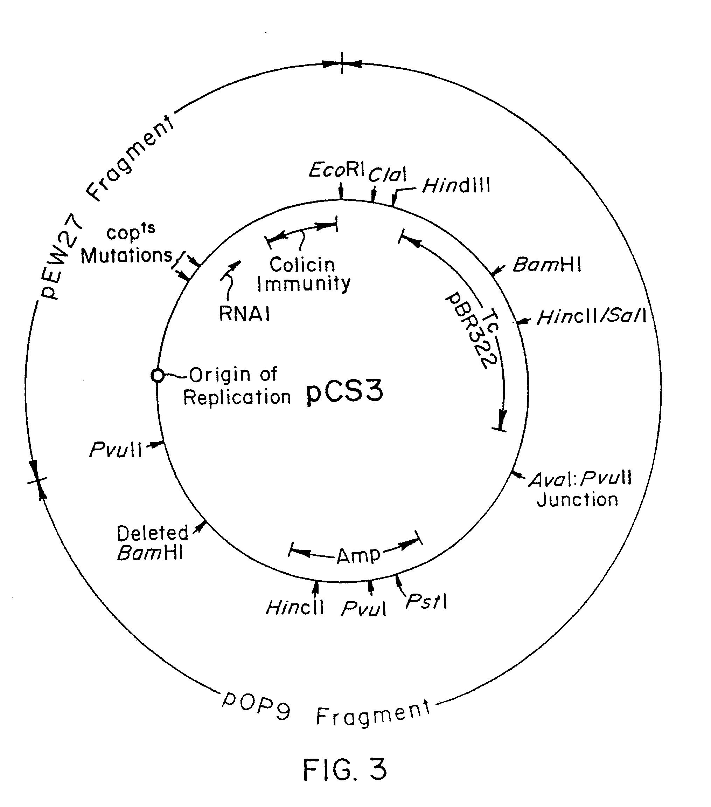

A particularly suitable construction in accordance with the invention is shown in the drawings in FIGURE 3. This construction has been derived from the two plasmids shown in FIGURES 1 and 2. The plasmid shown in FIGURE 1 is a ColEI derivative prepared as described below in accordance with known genetic engineering techniques. The plasmid shown in FIGURE 2, pEW27, is a plasmid shown and described by Wong, E.M., Muesing, M.A., Polisky, B., Proc. Natl. Acad. Sci. USA , 79:3570-3574 (1982). However, temperature-sensitive mutation carrying fragments (cop$^{ts}$) from other ColEI type plasmids may be substituted for the origin containing fragment of a ColEI plasmid vector in order to construct a plasmid in accordance with the invention.

The plasmid of the invention shown in FIGURE 3 was constructed by incorporating the fragment extending from the Eco RI site in the plasmid of FIGURE 1 clockwise to the Pvu II site. This fragment contains two regions encoding drug resistance: the tetracycline resistance derived from PBR322, and the gene for ampillin resistance derived from pOP6. However, the Tn3 transposon, in which the ampicillin resistance gene as well as the original BamHI site are originally contained, has been engineered (as described below) to have a deletion which removes the majority of the gene encoding the Tn3 transposase. Tn3 transposase is absolutely required for transposition. Heffron et al. (1979), Cell 18 , 1153. Thus, the stability of the plasmid is explainable by the loss of the Tn3 transposase.

The remaining portion of the plasmid in FIGURE 3 was provided from the plasmid in FIGURE 2 and was the fragment from the Pvu II site to the Eco RI site proceeding clockwise in pEW27. This fragment, which contains the gene for colicin resistance, contains two mutations providing the copy number temperature sensitivity characteristic. The resulting plasmid, shown in FIGURE 3, contains marker genes for tetracycline resistance and ampicillin resistance, and contains a number of convenient sites for insertion of heterologous DNA and accompanying promoters if desired.

In engineering a preferred form of the plasmid of the invention, a ColEI type plasmid, pOP6 or pOPI 6 was utilized. This plasmid is described in Gelfand, D.H., Shepard, H.M., O'Farrell, P.H., Polisky, B., Proc. Natl. Acad. Sci. USA , 75:5869-5873 (1978).

A 3.3 kb fragment of pOP6 was first deleted. To do this, 50 micrograms of pOP6 DNA was digested to completion with 20 units (15 min) each of restriction endonucleases Bam HI and Sst I in a 200 microliter reaction containing 10 mM Tris-Cl pH 7.5, 6 mM MgCl$_2$, 6 mM 2-mercaptoethanol, 100 micrograms/ml bovine serum albumin and 35 mM NaCl for two hours, 37°C. The digested DNA was phenol extracted, chloroform-isoamyl alcohol (24:1) extracted, ethanol precipitated, and resuspended in 10 mM Tris-Cl pH 7.4, 10 mM NaCl, 1 mM EDTA.

In order to eliminate to the Sst I 3' protruding ends and "fill in" or repair the Bam HI 5' ends, the digested pOP6 DNA was treated with E. coli DNA polymerase I. This was accomplished in a two stage reaction: the 1st stage at 20°C at which temperature the "3' -5' exo-" activity of the polymerase is expected to effectively eliminate the 3' Sst I protruding end and then a 0°C reaction in which the exonuclease activity is minimized and the polymerase activity repairs the now recessed 3' ends using the 5' template strand.

0.26 pMoles of Bam HI/Sst I digested pOP6 DNA was treated with 2 units E. coli DNA polymerase I in 50 microliters containing 500 pMoles of each deoxynucleotide triphosphate, 40 mM KPO$_4$ pH 7.4, 6.4 mM MgCl$_2$, 1 mM 2-mercaptoethanol and 100 micrograms/ml crystalline bovine serum albumin at 20°C for 10 minutes and then 0°C for an additional 15 minutes.

The polymerase treated DNA was deproteinized with phenol and ethanol precipitated.

In order to maximize intramolecular blunt-end ligation 0.1 pMole of the digested and repaired DNA was ligated in a 50 microliters reaction volume containing 50 mM Tris-Cl pH 7.5, 10 mM MgCl₂, 10 mM dithiothreitol, 100 micrograms/ml crystalline bovine serum albumin, 500 mM ATP and 0.1 units T4 DNA ligase at 20°C for 2 hours.

0.02 pMole (100 mg) of the ligated DNA was used to transform competent DG75 (O'Farrell, P.H., Polisky, B., Gelfand, D.H., J. Bacteriol . 134:645-654 (1978)). Transformants were selected on L plates containing 50 micrograms/ml ampicillin and screened for:

1) a 3.3 kb deletion;

2) loss of an Sst I restriction endonuclease site; and

3) presence of a newly formed Bam HI restriction endonuclease site.

One candidate, designated pOP7, was chosen and the Bam HI site in pOP7 was then deleted. In order to construct a more useful high copy number cloning vehicle, a DNA fragment encoding a tetracycline resistance (Tc$^R$) function from the plasmid pBR322 was ligated with a subfragment of pOp7. The Tc$^R$ fragment from pBR322 contains a Bam HI cleavage site. The single Bam HI recognition site in pOP7 was therefore eliminated by polymerase repair synthesis of the exposed 5' Bam HI cohesive ends.

25 micrograms of pOP7 DNA were digested to completion with 20 units Bam HI in 100 microliters containing 100 mM NaCl, 6 mM Tris-Cl pH 7.9, 6 mM 2-mercaptoethanol and 100 micrograms/ml bovine serumalbumin for 4-5 hours at 37°C. The digested DNA was deproteinized sequentially with phenol and chloroform-isoamyl alcohol (24:1) and precipitated with ethanol. The DNA was resuspended at 750 micrograms/ml in 10 mM Tris-Cl, pH 7.4, 1 mM EDTA.

In order to repair the 5' Bam HI cohesive ends 10 micrograms of Bam HI digested pOP7 DNA was treated for 1 hour at 37°C in 50 microliters containing 10 microMoles dTTP, dATP, dGTP and 80 pMoles ³H dCTP, 40 mM KPO₄ pH 7.5, 6.4 mM MgCl₂, 1 mM 2-mercaptoethanol, 100 micrograms/ml bovine serum albumin, and 0.7 units E. coli DNA polymerase I fragment A (Klenow). The DNA was deproteinized sequentially with phenol and chloroform isoamyl-alcohol (24:1) and concentrated by ethanol precipitation. The repaired and concentrated DNA was ligated in 10 microliters containing 0.5 micrograms (.1 pMole) DNA, 1 unit T4 DNA ligase, 1 mM ATP, 50 mM Tris-Cl pH 7.5, 10 mM MgCl₂, 10 mM dithriothreitol and 100 micrograms/ml bovine serum albumin at 20°C for 8-12 hours. Competent DG75 was treated with 0.1 micrograms of the DNA and transformants selected on plates containing 50 micrograms/ml ampicillin. Candidates were screened for the loss of the Bam HI restriction endonuclease cleavage

site. One candidate, designated pOP8, was selected.

This pOP8 was then modified to result in pOP9. To do this, the Ava I (repaired)-Eco RI Tc$^R$ fragment from pBR322 was first isolated. 100 micrograms of pBR322 were digested to completion in 500 microliters with 20 units Ava I in 60 mM NaCl, 6 mM Tris-Cl pH 8, 6 mM 2-mercaptoethanol, 10 mM MgCl₂ and 100 micrograms/ml bovine serum albumin for 3-1/2 hours at 37°C. The digested DNA was deproteinized and concentrated by ethanol precipitation to 2 micrograms/microliter.

To repair the Ava I ends of pBR322, 80 micrograms (55 pMoles of ends) of Ava I digested pBR322 was treated with 1.4 units E. coli DNA polymerase Klenow fragment in 200 microliters containing 40 mM KPO₄ pH 7.4, 6.6 mM MgCl₂, 10 uM each dTTP, dCTP, dATP, 1.09 nMoles ³H dGTP 1mM 2-mercaptoethanol and 100 micrograms/ml bovine serum albumin for 15 minutes, 20°C. The blunt-ended DNA was deproteinized and concentrated by ethanol precipitation to 1.6 micrograms/microliters. To liberate the Tc$^R$ fragment from pSR322, the flush-ended DNA was treated with Eco RI restriction endonuclease.

78 micrograms of DNA was digested to completion with 100 units Eco RI at 37°C for 1 hour in 100 microliters containing 100 mM Tris-Cl pH 7.5, 50 mM NaCl 5 mM MgCl₂ and 100 micrograms/ml gelatin. The smaller 1.4 kb fragment was separated from the larger fragment electrophoretically in a 0.6% agarose gel, eluted and the DNA concentrated to 0.2 micrograms/microliters (.2 pMoles/microliters).

Then, the Pvu II (partial)-Eco RI 3560 bp fragment from pOP8 was isolated. 95 micrograms pOP8 was partially digested in two separate reactions with 30 units (15 min) Pvu II in 600 microliters containing 6 mM Tris-Cl, pH 7.5, 60 mM NaCl, 6 mM MgCl₂, 6 mM 2-mercaptoethanol, and 100 micrograms/ml gelatin for 10 minutes at 37°C.

Following deproteinization with phenol and ethanol precipitation, each partial pOP8 Pvu II digest was digested to completion with Eco RI. 80 micrograms of DNA was treated with 100 units Eco RI in 200 microliters containing 100 mM Tris-Cl pH 7.5, 50 mM MgCl₂, 50mM NaCl, 100 micrograms/ml gelatin at 37°C for 75 min. The resulting digest was resolved electrophoretically and the appropriate 3.56 kb fragment isolated.

Ligation and transformation of 1.42 kb Eco RI-Ava I (repair) Tc$^R$ fragment (A) and 3.56 kb Eco RI-Pvu II Ap$^R$ fragment (B) was then accomplished. 0.5 micrograms of fragment B and 4.5 micrograms of fragment A were ligated in a two stage reaction in order to favor intermolecular ligation of the Eco RI ends. The 30 microliter reaction contained in addition to the DNA fragments 50 mM Tris-Cl pH 7.5, 10 mM MgCl$_2$, 10 mM dithiothreitol, 40 micro-Moles ATP and 1 microliter (about .001 unit) T4 DNA ligase. Ligation was initiated at 25°C and cooled gradually to 12°C over a period of 7 hours. ATP concentration was increased to 1 mM and about 0.1 unit T4 DNA ligase was added.

Competent DG75 was transformed with 5 microliters of the ligation mix, and transformants were selected on ampicillin (50 micrograms /ml) or ampicillin (50 micrograms /ml) and tetracycline (15 micrograms /ml) containing plates.

Candidates Tc$^R$ and Ap$^R$ colonies were screened for colicin immunity conferred by pOP8, size and restriction digestion pattern. One candidate which had:-

1) high copy number;
2) AP$^R$;
3) Tc$^R$;
4) Col immunity;
5) single restriction endonuclease sites for Eco RI, Bam HI, Pvu II, Hin dIII;
6) 2 restriction endonuclease sites for Hin cII; and
7) the appropriate size and Hae III digestion pattern, was designated pOP9 (FIGURE 2).

In order to accomplish conversion of pOP9 (co p⁻) (FIGURE 2) topCS3 (co p$^{ts}$)(FIGURE 3), a fragment from pEW27 was used. pEW27 is a nitrosoguanidine induced mutant plasmid derivative of pBGPI20 (Polisky, B., Bishop, R.J., Gelfand, D.H., Proc. Natl. Acad. Sci. USA , 73:3900-3904 (1976)). The plasmid contains a temperature-sensitive copy number (Cop$^{ts}$) alteration which increases plasmid copy number at elevated temperature. The plasmid phenotype was originally detected as an increase in beta-galactosidase synthesis in an F'lac I$^q$ host at 41°C (Gelfand, D. H., Shepard, H.M., O'Farrell, P.H., Polisky, B., Proc. Natl. Acad. Sci. USA, 75:5864-5873 (1978)) and increased beta-lactamase synthesis at 41°C (Muesing, M., Tamm, J., Shepard, H.M., Polisky, B., Cell, 24:235-242 (1981); O'Callaghan, C.H., Morris, A., Kirby, S.M., Shingler, A.H., Anti. Ag. Chemo. , 1:283-288 (1972). pEW27 contains a 2.06 kb Pvu II fragment that is homologous with the Pvu II A fragment of pOP8. The unique Eco RI site in pEW27 (and pBGPI20, Polisky, B., Biship, R.J., Gelfand, D.H., Proc. Natl Acad. Sci. USA, 73:3900-3904 (1976)) is in this fragment. The 1.6 kb Eco RI-Pvu II fragment of pEW27 is homologous with the 1.6 kb Eco RI-Pvu II origin containing fragment of pOP9 (cop⁻). To convert pOP9 (cop⁻) to pCS3, the 1.6 kb Eco RI-Pvu II fragments were switched.

50 micrograms pEW27 DNA was digested to completion with Pvu II and then Eco RI. DNA was digested for 90 minutes, 37°C in 100 microliters containing 6 mM Tris-Cl pH 7.5, 60 mM NaCal, 6 mM MgCl$_2$, 6 mM 2-mercaptoethanol, 100 micrograms/ml bovine serum albumin and 25 units (15 minutes) Pvu II. The Tris-Cl concentration was increased to 100 mM and Eco RI endonuclease was added (100 units/60 minutes ) for 90 min. The Eco RI-Pvu II 1.6 kb "E" fragment was electroeluted and concentrated by ethanol precipitation. Similarly, 50 micrograms of pOP9 was digested to completion with Pvu II and Eco RI and the 3.3 kb "A" fragment was isolated.

0.36 micrograms (.327 pMoles) pEW27 fragment and 0.35 micrograms (0.16 pMoles) pOP9 fragment were ligated in 20 microliter containing 50 mM Tris-Cl pH 7.5, 10 mM MgCl$_2$, 10 mM dithiothreitol, 1 mM ATP, 100 micrograms/ml bovine serum albumin and 0.1 unit T4 DNA ligase for 16 hours at 15°C. MM294 was transformed with 1/2 of the ligated DNA and ampicillin-tetracycline resistant transformants selected. Candidate colonies were initially screened at 30°C and 41°C on beta-lactamase assay plate and then for plasmid DNA levels following growth at 30°C and 41°C. One candidate was designated pCS3. Plasmid pCS3 is deposited with the American Type Culture Collection under accession number ATCC 39142.

The plasmids of the invention exist at a low copy number (15-20) at 30°C. At 40°C, even though the host cells themselves do not grow or replicate additionally, the plasmids multiply to 150-400 copies per chromosome per cell with consequent amplification of any products of heterologous genes cloned into such plasmids. The plasmid of the invention is relatively small in size and therefore less likely to suffer spontaneous deletions than larger plasmids.

As an example, the plasmids of the invention may be utilized in a process in which the trp promoter-beta interferon Eco RII/Xho II fragment existing in plasmid pBR322-BI trp is cloned into the Eco RI-Bam HI region of the Tc$^R$ gene in pCS3. The resulting overproduction of beta interferon (IFN-BI) is dramatic.

Transfer of E. coli trp promoter, operator, trpL, ribosome binding site and human IFN-BI structural gene from pBR322-BI trp to pCS3 is done as follows. The trp and IFN-BI sequences in pBR322 BI trp can be isolated as a Eco RI-Xh oII 620 bp DNA fragment. This DNA fragment is substituted for the 375 bp Eco RI-Bam HI DNA fragment in pCS3 utilizing the 4.6 kb origin and Ap$^R$ Bam HI-Eco RI "A" fragment. Ligation is achieved through homologous Eco RI ends (AATT) and Xho II/Bam HI ends (GATC).

The trp and IFN-BI sequences are first isolated. 50 micrograms of pBR322-BI-trp - were digested to completion in 200 microliters containing 10 mM Tris-Cl-pH 7.5, 6 mM MgCl$_2$, 6 mM 2-mercaptoethanol, 100 micrograms/ml gelatin and 50 units (60 minutes) of Xho II at 32°C for 60 minutes. The Tris-Cl concentration was increased to 100 mM, NaCl concentration increased to 50 mM and 100 units Eco RI added for 40 minutes at 37°C in order to convert 973 bp Xho II "C" fragment to the desired 620 bp Eco RI-Xho II "D" fragment. The desired fragment was electrophoretically fractionated and eluted through a 2% agarose gel and appropriate fractions concentrated by ethanol precipitation.

To prepare the pCS3 vector, 50 micrograms pCS3 were digested to completion with Bam HI (32 units-15 minutes) for 1 hour at 37°C in 100 microliters containing 10 mM Tris-Cl pH 7.5, 6 mM MgCl₂, 50 mM NaCl, 6 mM-2-mercaptoethanol and 100 micrograms/ml bovine serum albumin. The Tris-Cl concentration was increased to 100 mM and 100 units Eco RI added for 30 minutes at 37°C. The digested DNA was successively deproteinized with phenol and chloroform:isoamyl alcohol (24:1) and concentrated by ethanol precipitation.

Fragment ligation and transformation was the performed, 0.6 micrograms of digested vector and 0.2 micrograms of purified target fragment were ligated in 10 microliters containing 50 mM Tris-Cl pH 7.5, 10 mM MgCl₂, 10 mM dithiothroitol, 100 micrograms/ml bovine serum albumin and 0.001 unit T4 DNA ligase for 12 hours at 10°C. The ligated DNA was heated to 65° for 10 minutes, NaCl added to 100 mM and 3-units Bam HI added for 30 minutes at 37°C to inactivate (linearize) any vector ligation products that contained the original vector 375 bp Eco RI-Bam HI fragment. Competent DG75 was transformed with 1/5 of the ligated DNA and transformants selected on L plates containing 50 micrograms/ml ampicillin and pre-spread with 500 micrograms L-tryptophan.

Candidate colonies were screened for:
1) loss of TcR (substitution of trp -IFN beta-I sequences for a portion of the Tc gene);
2) correct plasmid size;
3) presence of unique Hpa I site (in trp promoter)
4) presence of additional Pst I site (in IFN-BI sequence 250 bp from unique Eco RI site)
5) presence and correct size of the 620 bp Eco RI-Xho II fragment that was originally purified from pBR322 beta-I trp . One correct candidate was designated pCS3 beta-I trp or pCS4.

The plasmids of the invention, because of their ColEI type parentage, have an absolute requirement for the enzyme DNA polymerase I and will not replicate in a Pol AI- (DNA polymerase I lacking) host (Kingsbury D.T., Helinski, D.R., Biochem. Biophys, Res. Comm., 41:1538-1544 (1970)); Falkow, S., Infectious Multiple Drug Resistance, Pion Limited, London, pp. 151-152 (1975)). The plasmids characteristically exist at 15-30 copies per chromosome per cell and may be amplified by the addition of chloramphenicol, spectinomicin, etc. (certain antibiotics that inhibit protein synthesis) Clewell, D. B., J. Bacteriol. 110:667-676 (1972). The very high copy number characteristic of the plasmids of the invention is not substantially altered and, if anything, it is slightly increased by the addition of chloremphenicol. This indicates that there is no requirement for protein synthesis in order for amplification to take place.

It may be seen, therefore, that the invention provides stable high copy number temperature sensitive plasmids with ColEI type parentage. The plasmids are temperature sensitive relatively small in size, and a substantial amplification of copy number occurs upon a temperature shift from, for example, 30°C to 40°C.

Various modifications within the scope of the invention in addition to those shown and described herein will become apparent to those skilled in the art from the foregoing description and accompanying drawings.

## Claims

1. A stable recombinant plasmid having the following characteristics:

   a. the plasmid comprises a region derived from a colicin EI (Col EI)-type plasmid, wherein said region causes intracellular replication of the plasmid to undergo an increase upon an increase in temperature, wherein said increase in replication is independent of protein synthesis and is RNA-controlled;

   b. the plasmid lacks an active transposon 3 (Tn3);

   c. the plasmid comprises two regions each encoding drug resistance to a different drug, one of the regions containing a restriction enzyme site positioned such that insertion of a DNA sequence into said site causes loss of the respective drug resistance; and

   d. after insertion of a DNA sequence into the plasmid expressibly encoding a foreign polypeptide the resulting plasmid is stable during expression of the encoded foreign polypeptide when the copy number of the plasmid has been increased.

2. A plasmid as claimed in claim 1, wherein the increase in temperature is from about 30°C to about 40°C.

3. A plasmid as claimed in claim 1 or claim 2 less than about 6 kilobases in size and containing the PvuII/EcoRI fragment of plasmid pEW27.

4. Plasmid pCS3 (obtainable from ATCC 39142).

5. An expression plasmid comprising a plasmid as claimed in any one of Claims 1 to 4 and a DNA sequence defining a foreign polypeptide which has been inserted into the site defined in (c) of Claim 1.

6. A process for obtaining a plasmid as defined in claim 1, which process comprises ligating fragments together to produce an engineered plasmid of ColEI parentage, which lacks an active transposon 3 (Tn3), one of the fragments having been obtained by enzymatically digesting a plasmid, preferably plasmid pEW27, containing a temperature-sensitive copy number portion which increases plasmid copy number at elevated temperature, to produce a fragment containing said portion.

7. A process for obtaining an expression plasmid comprising either (a) carrying out a process as defined in claim 6, in which one of the said fragments contains a DNA sequence which is encoded for a heterologous gene product in the host; or (b) obtaining a plasmid by a process as defined in claim 6 and then ligating a fragment containing a DNA sequence which is encoded for a heterologous gene product in the host into a site in the resulting plasmid produced by enzyme digestion thereof.

8. A microorganism containing a plasmid as claimed in any one of claims 1 to 5

9. A process for obtaining a microorganism as defined in claim 8 which comprises transforming host microorganism cells with a plasmid as defined in any one of claims 1 to 5.

10. A process for preparing a protein product which comprises cultivating a host microorganism containing as part of its phenotypically expressible genotype a plasmid as defined in claim 1 and which includes a portion encoded for production of said protein product, and allowing the microorganism phenotypically to express its genotype including said plasmid.

**Revendications**

1. Plasmide recombinant stable ayant les caractéristiques suivantes :
   a. le plasmide comprend une région dérivée d'un plasmide de type colicine EI (ColEI), cette région provoquant une augmentation de la réplication intracellulaire du plasmide lorsque la température s'élève, cette augmentation de la réplication étant indépendante de la synthèse des protéines et régulée par l'ARN ;
   b. le plasmide est dépourvu d'un transposon 3 (Tn) actif ;
   c. le plasmide comprend deux régions codant chacune pour la résistance à une drogue différente, une des régions contenant un site d'enzyme de restriction disposé de telle sorte que l'insertion d'une séquence d'ADN dans ce site provoque la perte de la résistance à la drogue correspondante ; et
   d. après insertion dans le plasmide d'une séquence d'ADN codant pour un polypeptide étranger et pouvant l'exprimer, le plasmide obtenu est stable pendant l'expression du polypeptide étranger pour lequel il code lorsque le nombre des copies du plasmide a été augmenté.

2. Plasmide selon la revendication 1, pour lequel l'élévation de la température est d'environ 30° C à environ 40° C.

3. Plasmide selon la revendication 1 ou la revendication 2, dont la taille est inférieure à environ 6 kilobases et contenant le fragment Pvu II/EcoR I du plasmide pEW27.

4. Plasmide pCS3 (pouvant être obtenu à partir d'ATCC 39142).

5. Plasmide d'expression comprenant un plasmide selon l'une quelconque des revendications 1 à 4 et une séquence d'ADN définissant un polypeptide étranger qui a été insérée dans le site défini en (c) de la revendication 1.

6. Procédé pour obtenir un plasmide selon la revendication 1, qui comprend la ligature mutuelle de fragments pour produire un plasmide de génie génétique ayant une ascendance de type colEI, dépourvu d'un transposon 3 (Tn3) actif, un des fragments ayant été obtenu par digestion enzymatique d'un plasmide, de préférence le plasmide pEW27, contenant une portion de thermosensibilité du nombre des copies qui accroît le nombre des copies du plasmide à température élevée, pour produire un fragment contenant cette portion.

7. Procédé pour obtenir un plasmide d'expression comprenant soit (a) la réalisation d'un procédé comme défini dans la revendication 6, dans lequel un desdits fragments contient une séquence d'ADN qui code pour un produit de gène hétérologue chez l'hôte ; soit (b) l'obtention d'un plasmide selon un procédé défini dans la revendication 6, puis la ligature d'un fragment contenant une séquence d'ADN qui code pour un produit de gène hétérologue dans l'hôte dans un site du plasmide obtenu produit par la digestion enzymatique de celui-ci.

8. Microorganisme contenant un plasmide selon l'une quelconque des revendications 1 à 5.

9. Procédé pour l'obtention d'un microorganisme selon la revendication 8, qui comprend la transformation des cellules d'un microorganisme hôte avec un plasmide selon l'une quelconque des revendications 1 à 5.

10. Procédé pour préparer un produit protéinique, qui consiste à cultiver un microorganisme hôte contenant, comme partie de son génotype susceptible d'expression phénotypique, un plasmide selon la revendication 1 et qui comprend une portion codant pour la production dudit produit protéinique, et permettre, audit microorganisme, l'expression phénotypique de son génotype comprenant ledit plasmide.

**Ansprüche**

1. Stabiles rekombinantes Plasmid mit den folgenden Merkmalen:
   a. das Plasmid enthält einen von einem Colicin-EI(ColEI)-Typ-Plasmid abgeleiteten Bereich, wobei der Bereich bewirkt, daß die intrazelluläre Replikation des Plasmids bei Erhöhung der Temperatur zunimmt, wobei die Zunahme der Replikation unabhängig von der Proteinsynthese und RNA-kontrolliert ist;
   b. dem Plasmid fehlt ein aktives Transposon 3 (Tn3);
   c. das Plasmid enthält zwei Bereiche, die jeder die Arzneistoffresistenz gegen ein unterschiedliches Arzneimittel codieren, wobei einer der Bereiche eine Restriktionsenzymstelle enthält, die so liegt, daß die Insertion einer DNA-Sequenz an dieser Stelle den Verlust der entsprechenden Arzneimittelresistenz bewirkt; und
   d. nach Insertion einer DNA-Sequenz in das Plasmid, die ein fremdes Polypeptid exprimierbar codiert, ist das entstehende Plasmid während der Expression des codierten fremden Polypeptids stabil, wenn die Kopienzahl des Plasmids erhöht worden ist.

2. Plasmid nach Anspruch 1, wobei die Temperaturerhöhung von etwa 30°C auf etwa 40°C stattfindet.

3. Plasmid nach Anspruch 1 oder Anspruch 2, dessen Größe weniger als etwa 6 Kilobasen beträgt und das das PvuII/EcoRI-Fragment des Plasmids pEW27 enthält.

4. Plasmid pCS3 (erhältlich unter ATCC 39142).

5. Expressionsplasmid, enthaltend ein Plasmid nach einem der Ansprüche 1 bis 4 und eine ein fremdes Polypeptid definierende DNA-Sequenz, die in die in (c) von Anspruch 1 definierte Stelle eingefügt worden ist.

6. Verfahren zum Erhalt eines Plasmids nach Anspruch 1, wobei das Verfahren die Ligierung von Fragmenten zur Herstellung eines von ColEI abgeleiteten gentechnischen Plasmids umfaßt, dem ein aktives Transposon 3 (Tn3) fehlt, wobei eines der Fragmente durch enzymatische Spaltung eines Plasmids, vorzugsweise des Plasmids pEW27, erhalten worden ist, das einen temperaturempfindlichen Kopienzahl-Bereich enthält, der die Kopienzahl des Plasmids bei erhöhter Temperatur steigert, zur Herstellung eines den Bereich enthaltenden Fragments.

7. Verfahren zum Erhalt eines Expressionsplasmids, umfassend entweder (a) Durchführung eines Verfahrens nach Anspruch 6, wobei eines der Fragmente eine DNA-Sequenz enthält, die im Wirt für ein heterologes Genprodukt codiert; oder (b) Erhalt eines Plasmids durch ein Verfahren nach Anspruch 6 und anschließend Ligierung eines Fragments, enthaltend eine DNA-Sequenz, die im Wirt für ein heterologes Genprodukt codiert, in eine durch Enzymspaltung des Plasmids entstehende Stelle.

8. Mikroorganismus, der ein Plasmid nach einem der Ansprüche 1 bis 5 enthält.

9. Verfahren zum Erhalt eines Mikroorganismus nach Anspruch 8, das die Transformation von Wirtsmikroorganismus-Zellen mit einem Plasmid nach einem der Ansprüche 1 bis 5 umfaßt.

10. Verfahren zur Herstellung eines Proteinproduktes, umfassend die Züchtung eines Wirtsmikroorganismus, der als Teil seines phänotypisch exprimierbaren Genotyps ein Plasmid nach Anspruch 1 enthält, das einen für die Herstellung des Proteinproduktes codierten Bereich umfaßt, und die phänotypische Expression des Genotyps, einschließlich des Plasmids, durch den Mikroorganismus.

FIG. I

FIG. 2

FIG. 3